Europäisches Patentamt

European Patent Office

Office européen des brevets

(19)

(11) Veröffentlichungsnummer: **0 259 346**
**B1**

(12) EUROPÄISCHE PATENTSCHRIFT

(45) Veröffentlichungstag der Patentschrift: **04.04.90**

(51) Int. Cl.⁵: **C 07 C 255/24**

(21) Anmeldenummer: **86905771.1**

(22) Anmeldetag: **05.09.86**

(86) Internationale Anmeldenummer:
**PCT/EP86/00513**

(87) Internationale Veröffentlichungsnummer:
**WO 87/05618 24.09.87 Gazette 87/21**

(54) **r-1-CYAN-1-R2 -CIS-4-R1 -CYCLOHEXANVERBINDUNGEN.**

(30) Priorität: **14.03.86 DE 3608500**

(43) Veröffentlichungstag der Anmeldung:
**16.03.88 Patentblatt 88/11**

(45) Bekanntmachung des Hinweises auf die
Patenterteilung:
**04.04.90 Patentblatt 90/14**

(84) Benannte Vertragsstaaten:
**CH DE FR GB LI SE**

(56) Entgegenhaltungen:
EP-A-0 107 759
WO-A-86/06401
WO-A-86/07055
GB-A-1 229 400

Chemical Abstracts, vol. 90, No. 11, 12 March
1979 (Columbus, Ohio, USA), A. A. Agekyan et
al. "Arylalkylamine derivatives. XIV. Synthesis
of some cycloalkane-substituted
arylalkylamines" see page 572, abstract No.
86895p, Arm. Khim.Zh.1978, 31(9), 689-93

(73) Patentinhaber: **MERCK PATENT GESELLSCHAFT
MIT BESCHRÄNKTER HAFTUNG**
**Frankfurter Strasse 250 Postfach 4119**
**D-6100 Darmstadt (DE)**

(72) Erfinder: **WÄCHTLER, Andreas**
**Goethestrasse 34**
**D-6103 Griesheim (DE)**
Erfinder: **KRAUSE, Joachim**
**Samuel-Morse-Str. 14**
**D-6110 Dieburg (DE)**
Erfinder: **EIDENSCHINK, Rudolf**
**Konrad Adenauer Strasse 1**
**D-6109 Mühltal 1 (DE)**
Erfinder: **HITTICH, Reinhard**
**Am Kirchberg 11**
**D-6101 Modautal 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die Erfindung betrifft r-1-Cyan-1-R²-cis-4-R¹-cyclohexanverbindungen, die sich insbesondere als Zwischenprodukte zur Herstellung von Flüssigkristallverbindungen eignen, sowie ihre Herstellung.

Die erfindungsgemäßen Cyclohexanverbindungen können wie ähnliche, nicht durch CN substituierte Verbindungen, z.B. wie die in der DE—OS 28 00 553 aufgeführten Verbindungen, als Zwischenprodukte für die Synthese flüssigkristalliner Materialien verwendet werden.

Der Erfindung lag die Aufgabe zugrunde, Cyclohexanderivate und ihre Herstellung anzugeben, die sich zur besonders einfachen Synthese von Flüssigkristallverbindungen mit negativer dielektrischer Anisotropie eignen.

Es wurde nun gefunden, daß die erfindungsgemäßen Cyclohexanverbindungen vorzüglich zur einstufigen Synthese einer Vielzahl von Ziehlprodukten geeignet sind.

Mit der Bereitstellung der erfindungsgemäßen Cyclohexanverbindungen wird außerdem ganz allgemein die Palette der Zwischenprodukte, die sich unter vershiedenen Gesichtspunkten zur Herstellung von Flüssigkristallen eignen, erheblich verbreitert.

Gegenstand der Erfindung sind somit die r-1-Cyan-1-R²-cis-4-R¹-cyclohexanverbindungen, worin R¹ —CH₂X, Amino, Amidino, Formyl, p-R³-Phenyl oder 4-R³-Biphenyl-4'-yl ist, wobei X Hydroxy, Halogen oder eine Sulfonatgruppe und R³ Amino, Amidino, Formyl, Hydroxy, Carboxy, Alkanoyl mit 2 bis 12 C-Atomen oder —CH₂X, worin die oben angegebene Bedeutung hat, bedeutet, und R² eine Alkylgruppe mit 1 bis 12 C-Atomen ist, worin auch eine oder mehrere nicht benachbarte und nicht ringständige CH₂-Gruppen durch —O— oder —CH=CH— und/oder eine oder mehrere CH₂-Gruppen durch —CF₂— und/oder —CHF— ersetzt sein können, deren Herstellung sowie deren Verwendung als Zwischenprodukte bei der Herstellung von Flüssigkristallmaterialien.

Die erfindungsgemäßen Cyclohexanverbindungen sowie deren reaktionsfähige Derivate lassen sich erfindungsgemäß als Zwischenprodukte zur Herstellung von Flüssigkristallmaterialien verwenden, wobei sie beispielsweise mit in der Flüssigkristallchemie bekannten Carbonsäuren, Phenolen oder Alkoholen verethert oder verestert werden können oder durch Umsetzung mit bekannten metallorganischen Verbindungen zum Beispiel in entsprechende Ethanderivate überführbar sind.

Als reaktionsfähige Derivate der erfindungsgemäßen Verbindungen eignen sich in erster Linie Säurehalogenide, Phenolate oder Alkoholate; als Säureadditionsalze sind beispielsweide die Hydrochloride der Amidinoverbindungen von Interesse.

Die erfindungsgemäßen Cyclohexanverbindungen können nach an sich bekannten Methoden hergestellet werden, wie sie in der Literatur (z.B. in en Standardwerken wie Houben-Weyl, Methoden der organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen. Alle Ausgangsmaterialien sind literaturbekannt oder können nach literaturbekannten Methoden hergestellt werden.

Bevorzugte Ausgangsmaterialien sind die im Handel erhältlichen bzw. literaturbekannten Chemikalien trans-4-Cyancyclohexancarbonsäure (CA *70*, 37 293 u (1969)), trans-4-Cyancyclohexanol, trans-4-Phenylcyclohexancarbonitril, trans-4-(p-Methoxyphenyl)-cyclohexancarbonitril, trans-4-(Biphenyl-4'-yl)-cyclohexancarbonitril (erhältlich analog trans-4-Phenylcyclohexancarbonitril) und trans 4-(4-Alkoxy-biphenyl-4'-yl)-cyclohexancarbonitril (DE—OS 27 01 591), welche sich nach literaturbekannten Methoden zu den erfindungsgemäßen Cyclohexanverbindungen umsetzen lassen.

Diese Ausgangsmaterialien lassen sich durch Alkylierung in Gegenwart einer Base wie z.B. NaNH₂ oder Lithiumdiisopropylamid in die entsprechenden 1-Cyan-1-R²-cyclohexanderivate überführen, die gegebenenfalls durch weitere literaturbekannte Umsetzungen wie Etherspaltung, Friedel-Crafts-Acylierung und gegebenenfalls Haloformabbau o.ä. unter üblichen Reaktionsbedingungen in die erfindungsgemäßen Zwischenprodukte überführt werden können.

Die erhaltenen Carbonsäuren können beispielsweise nach Veresterung mit Ethanol mit LiCNBH₃ zu den Hydroxymethylverbindungen reduziert werden, die nach üblichen Verfahren in die entsprechenden Halogen- oder Sulfonatverbindungen überführt werden können. Die Jodmethylverbindungen, z.B. 4-Cyan-4-alkyl-1-jodmethylcyclohexane, sind auch durch Umsetzung der Mesylate mit Natriumjodid in Aceton zugänglich.

Die erfindungsgemäßen Amine sind z.B. aus den Carbonsäuren bzw. deren Amiden nach literaturbekannten Standardverfahren (z.B. Schmidt- oder Hoffmann-Umlagerung) oder aus den Ketonen bzw. deren Oximen durch Beckmann-Umlagerung und Verseifung erhältlich.

Die erfindungsgemäßen Cyclohexanderivate können geradkettige oder verzweigte Gruppen R² haben. Verbindungen mit verzweigten Gruppen können in Form des Racemates oder als optisch aktive Verbindungen eingesetzt werden.

R¹ ist vorzugsweise —CH₂OH, —CH₂Cl, —CH₂Br, —CH₂J oder insbesondere bevorzugt 4-Hydroxybiphenyl-4'-yl. R³ ist vorzugsweise Hydroxy oder Carboxy, ferner Alkanoyl mit vurzugsweise 2 bis 5 C-Atomen. R² ist vorzugsweise eine geradkettige Alkylgruppe mit 4 bis 12 C-Atomen, worin auch eine oder zwei nicht ringständige und nicht benachbarte CH₂-Gruppen durch —O— und/oder —C=CH— ersetzt sein können. R² bedeutet beispielsweise bevorzugt Butyl, Pentyl, Hexyl, Heptyl, Octyl, Nonyl, Decyl, Undecyl oder Dodecyl, ferner Methyl, Ethyl, Propyl, 2-, 3- oder 4-Oxapentyl, 2-, 3-, 4- oder 5-Oxahexyl, 2-, 3-, 4-, 5-oder 6-Oxaheptyl, 2-, 3-, 4-, 5-, 6- oder 7-Oxaoctyl, 2-, 3-, 4-, 5-, 6-, 7- oder 8-Oxanonyl, 2-, 3-, 4-, 5-, 6-, 7-, 8- oder 9-Oxadecyl, 1,3-Dioxabutyl (= Methoxymethoxy), 1,3-, 1,4- oder 2,4-Dioxapentyl, 1,3-, 1,4-, 1,5-, 2,4-, 2,5- oder 3,5-Dioxahexyl, 1,3-, 1,4-, 1,5-, 1,6-, 2,4-, 2,5-, 2,6-, 3,5-, 3,6- oder 4,6-Dioxaheptyl, 1,4-Dioxaoctyl, 1,4,7-Trioxaoctyl, 1,4-Dioxanonyl, 1,4-Dioxadecyl.

Bevorzugte verzweigte Reste $R^2$ sind Isopropyl, 2-Butyl (= 1-Methylpropyl), Isobutyl (= 2-Methylpropyl), 2-Methylbutyl, Isopentyl (= 3-Methylbutyl), 2-Methylpentyl, 3-Methylpentyl, 2-Ethylhexyl, 2-Propylpentyl, 2-Octyl, 2-Oxa-3-methylbutyl, 3-Oxa-4-methylpentyl, 2-Methyl-3-oxa-pentyl, 2-Methyl-3-oxahexyl, 2-Methyl-3-oxa-heptyl, 2-Methyl-3-oxa-octyl, 2-Methyl-3-oxa-nonyl und 2-Methyl-3-oxa-decyl.

$R^2$ ist ferner bevorzugt Perfluoralkyl, —$CH_2$-Perfluoralkyl oder —$CH_2CH_2$-Perfuoralkylmit jeweils 3 bis 12, vorzugsweise 5 bis 10, C-Atomen. Im Perfluoralkylteil können auch eine oder mehrere nicht benachbarte $CF_2$-Gruppen, insbesondere auch endständige Gruppen, durch CHF ersetzt sein.

Besonders bevorzugt sind r-1-Cyan-1-$R^2$-cis-4-$R^1$-cyclohexanverbindungen, worin $R^1$ —$CH_2X$, vorzugsweise —$CH_2Br$ oder —$CH_2J$, bedeutet. Weitere bevorzugte Bedeutungen von $R^1$ sind p-$R^3$-Phenyl und 4-$R^3$-Biphenyl-4-yl, wobei $R^3$ vorzugsweise Hydroxy, Carboxy, Alkanoyl oder Amidino, insbesondere bevorzugt Hydroxy, bedeutet.

Besonders bevorzugt sind Verbindungen nach Anspruch 1, worin $R^1$ p-Hydroxyphenyl oder 4-Hydroxybiphenyl-4'-yl ist. Diese Verbindungen sind aus den entsprechenden, bekannten p-Alkyloxyphenyl- bzw. 4-Alkyloxybiphenyl-4'-yl-Verbindungen durch Etherspaltung nach literaturbekannten Methoden erhältlich. Vorzugsweise wird die Etherspaltung, die ebenfalls Gegenstand dieser Erfindung ist, in Gegenwart einer Base wie Z.B. Kalium-tert.-butylat durchgeführt. Es können jedoch auch andere Basen oder Säuren wie z.B. Bortrichlorid oder Jod- oder Chlortrimethylsilan in Analogie zu literaturbekannten Verfahren eingesetzt werden.

Die Herstellung von Flüssigkristallmaterialien aus den erfindungsgemäßen Zwischenprodukten erfolgt nach an sich bekannten Methoden, wie sie in der Literatur (z.B. in den Standartwerken wie Houben-Weyl, Methoden der organischen Chemie, Georg-Thieme-Verlag, Stuttgart) beschrieben sind, und zwar unter Reaktionsbedingungen, die für die entsprechenden Umsetzungen bekannt und geeignet sind. Dabei kann man auch von an sich bekannten, hier nicht näher erwähnten Varianten Gebrauch machen. Aus den erfindungsgemäßen Zwischenprodukten herstellbare Flüssigkristallmaterialien sind beispielsweise beschrieben in DE—OS 36 08 500.

Die folgenden Beispiele sollen die Erfindung erläutern. Vor- und nachstehend bedeuten Prozentangaben Gewichtzprozent; alle Temperatur sind in Grad Celsius angegeben.

"Übliche Aufarbeitung" bedeutet: Man gibt Wasser hinzu, extrahiert mit Methylenchlorid, trennt ab, trocknet die organische Phase, dampft ein und reinigt das Produkt durch Kristallisation und/oder Chromatographie.

Beispiel 1

Zu einer aus 187 g 4-Cyan-4-n-heptyl-1-phenylcyclohexan (erhältylich durch Überführung von 4-Phenylcyclohexancarbonsäure in 4-Phenylcyclohexancarbonitril und anschließende Alkylierung mit n-Heptylbromid in Gegenwart von Lithiumdiisopropylamid), 130 ml Dichlormethan und 89 g Aluminiumchlorid erhältlichen Lösung wird bei 10° der aus 89 g Aluminiumchlorid, 57,4 ml Acetylchlorid und 330 ml Dichlormethan erhaltene Säurechloridkomplex zugetropft. Nach einer Stunde wird auf ein Gemisch von 1 kg Eis und 200 ml konzentrierte Salzsäure gegossen und gerührt. Die hellgelbe Mischung wird mit Dichlormethan extrahiert. Die vereinigten organischen Phasen werden mit Wasser, $NaHCO_3$-Lösung und anschließend mit Wasser neutral gewaschen, getrocknet und vom Lösungsmittel befreit. Nach Umkristallisation aus Hexan erhält man 4-(4-Cyan-4-n-heptylcyclohexyl)-acetophenon.

Analog werden hergestellt:
4-(4-Cyan-4-hexylcyclohexyl)-acetophenon
4-(4-Cyan-4-heptylcyclohexyl)-acetophenon
4-(4-Cyan-4-octylcyclohexyl)-acetophenon
4-(4-Cyan-4-nonylcyclohexyl)-acetophenon
4-(4-Cyan-4-decylcyclohexyl)-acetophenon

4-(4-Cyan-4-heptylcyclohexyl)-propionylbenzol
4-(4-Cyan-4-heptylcyclohexyl)-butyrylbenzol
4-(4-Cyan-4-heptylcyclohexyl)-pentanoylbenzol
4-(4-Cyan-4-heptylcyclohexyl)-hexanoylbenzol
4-(4-Cyan-4-heptylcyclohexyl)-heptanoylbenzol
4-(4-Cyan-4-heptylcyclohexyl)-octanoylbenzol

4-(4-Cyan-4-heptylcyclohexyl)-4'-acetylbiphenyl
4-(4-Cyan-4-heptylcyclohexyl)-4'-propionylobiphenyl
4-(4-Cyan-4-heptylcyclohexyl)-4'-butyrylbiphenyl
4-(4-Cyan-4-heptylcyclohexyl)-4'-pentanoylbiphenyl
4-(4-Cyan-4-heptylcyclohexyl)-4'-hexanoylbiphenyl
4-(4-Cyan-4-heptylcyclohexyl)-4'-heptanoylbiphenyl
4-(4-Cyan-4-heptylcyclohexyl)-4'-octanoylbiphenyl

Beispiel 2

Zu einer Lösung von 28 g Natriumhydroxid und 33,6 g Brom in 160 ml Wasser wird bei Raumtemperatur unter Rühren eine Lösung von 20 g 4-(4-Cyan-4-n-pentylcyclohexyl)acetophenon in 70 ml Dioxan getropft. Anschließend wird das Reaktionsgemisch noch 1 Stunde gerührt, mit einer Lösung von 10 g Natriumhydrogensulfit in 100 ml Wasser versetzt und mit Salzsäure auf einen pH-Wert von etwa eingestellt. Aus der wäßrigen Lösung wird durch zweimaliges Extrahieren mit je 100 ml Dichlormethan, Waschen der Extrakte mit Wasser und Eindampfen 4-(4-Cyan-4-n-pentylcyclohexyl)-benzoesäure isoliert.

Analog werden hergestellt:

4-(4-Cyan-4-methylcyclohexyl)-benzoesäure
4-(4-Cyan-4-ethylcyclohexyl)-benzoesäure
4-(4-Cyan-4-propylcyclohexyl)-benzoesäure
4-(4-Cyan-4-butylcyclohexyl)-benzoesäure
4-(4-Cyan-4-hexylcyclohexyl)-benzoesäure
4-(4-Cyan-4-heptylcyclohexyl)-benzoesäure, F. 154°
4-(4-Cyan-4-octylcyclohexyl)-benzoesäure
4-(4-Cyan-4-nonylcyclohexyl)-benzoesäure
4-(4-Cyan-4-decylcyclohexyl)-benzoesäure
4-(4-Cyan-4-methoxymethylcyclohexyl)-benzoesäure
4-(4-Cyan-4-methoxyethylcyclohexyl)-benzoesäure
4-(4-Cyan-4-ethoxyethylcyclohexyl)-benzoesäure
4-(4-Cyan-4-propoxycyclohexyl)-benzoesäure
4-(4-Cyan-4-trans-3-pentenylcyclohexyl)-benzoesäure

4-[p-(4-Cyan-4-methylcyclohexyl)-phenyl]-benzoesäure
4-[p-(4-Cyan-4-ethylcyclohexyl)-phenyl]-benzoesäure
4-[p-(4-Cyan-4-propylcyclohexyl)-phenyl]-benzoesäure
4-[p-(4-Cyan-4-butylcyclohexyl)-phenyl]-benzoesäure
4-[p-(4-Cyan-4-pentylcyclohexyl)-phenyl]-benzoesäure
4-[p-(4-Cyan-4-hexylcyclohexyl)-phenyl]-benzoesäure
4-[p-(4-Cyan-4-heptylcyclohexyl)-phenyl]-benzoesäure
4-[p-(4-Cyan-4-octylcyclohexyl)-phenyl]-benzoesäure
4-[p-(4-Cyan-4-nonylcyclohexyl)-phenyl]-benzoesäure
4-[p-(4-Cyan-4-decylcyclohexyl)-phenyl]-benzoesäure

Beispiel 3

Ein Gemisch von 13,7 g 4-(4-Cyan-4-octylcyclohexyl)-4'-octoxybiphenyl, 7,4 g Kalium-tert-butylat und 70 ml N-Methylpyrrolidon-2-wird unter einer Stickstoffatmosphäre 15 Stunden bei 150° gerührt. Die nach Zugabe des erhaltenen Gemisches zu Wasser erhaltene Suspension wird mit so viel Tetrahydrofuran versetzt, daß eine klare Lösung entsteht. Nach Ansäuern mit HCl wird mit Methylenchlorid extrahiert. Nach üblicher Aufarbeitung erhält man 4-(4-Cyan-4-octyl-cyclohexyl)-4'-hydroxyphenyl.

Analog werden hergestellt:

4-(4-Cyan-4-decylcyclohexyl)-4'-hydroxybiphenyl
4-(4-Cyan-4-nonylcyclohexyl)-4'-hydroxybiphenyl
4-(4-Cyan-4-heptylcyclohexyl)-4'-hydroxybiphenyl
4-(4-Cyan-4-hexylcyclohexyl)-4'-hydroxybiphenyl
4-(4-Cyan-4-pentylcyclohexyl)-4'-hydroxybiphenyl
4-(4-Cyan-4-butylcyclohexyl)-4'-hydroxybiphenyl
4-(4-Cyan-4-propylcyclohexyl)-4'-hydroxybiphenyl
4-(4-Cyan-4-perfluoropropylcyclohexyl)-4'-hydroxybiphenyl
4-(4-Cyan-4-perfluorbutylcyclohexyl)-4'-hydroxybiphenyl
4-(4-Cyan-4-perfluorpentylcyclohexyl)-4'-hydroxybiphenyl
4-(4-Cyan-4-perfluorhexylcyclohexyl)-4'-hydroxybiphenyl
4-(4-Cyan-4-perfluorheptylcyclohexyl)-4'-hydroxybiphenyl
4-(4-Cyan-4-perfluoroctylcyclohexyl)-4'-hydroxybiphenyl
4-(4-Cyan-4-perfluorethylmethylcyclohexyl)-4'-hydroxybiphenyl
4-(4-Cyan-4-perfluorpropylmethylcyclohexyl)-4'-hydroxybiphenyl
4-(4-Cyan-4-perfluorbutylmethylcyclohexyl)-4'-hydroxybiphenyl
4-(4-Cyan-4-perfluorpentylmethylcyclohexyl)-4'-hydroxybiphenyl
4-(4-Cyan-4-perfluorhexylmethylcyclohexyl)-4'-hydroxybiphenyl
4-(4-Cyan-4-perfluorheptylmethylcyclohexyl)-4'-hydroxybiphenyl
p-(4-Cyan-4-decylcyclohexyl)-phenol
p-(4-Cyano-4-nonylcyclohexyl)-phenol
p-(4-Cyano-4-heptylcyclohexyl)-phenol
p-(4-Cyano-4-hexylcyclohexyl)-phenol
p-(4-Cyano-4-pentylcyclohexyl)-phenol

p-(4-Cyano-4-butylcyclohexyl)-phenol
p-(4-Cyano-4-propylcyclohexyl)-phenol
p-(4-Cyano-4-perfluorpropylcyclohexyl)-phenol
p-(4-Cyano-4-perfluorbutylcyclohexyl)-phenol
p-(4-Cyano-4-perfluorpentylcyclohexyl)-phenol
p-(4-Cyano-4-perfluorhexylcyclohexyl)-phenol
p-(4-Cyano-4-perfluorheptylcyclohexyl)-phenol
p-(4-Cyano-4-perfluoroctylcyclohexyl)-phenol
p-(4-Cyano-4-perfluorethylmethylcyclohexyl)-phenol
p-(4-Cyano-4-perfluorpropylmethylcyclohexyl)-phenol
p-(4-Cyano-4-perfluorbutylmethylcyclohexyl)-phenol
p-(4-Cyano-4-perfluorpentylmethylcyclohexyl)-phenol
p-(4-Cyano-4-perfluorhexylmethylcyclohexyl)-phenol
p-(4-Cyano-4-perfluorheptylmethylcyclohexyl)-phenol

Beispiel 4

In ein Gemisch aus 19 g p-(4-Cyan-4-heptylcyclohexyl)benzonitril (erhältlich aus der entsprechenden Benzoesäure über das Säurechlorid, welches nach Überführung in das Säureamid mit Thionylchlorid behandelt wird), 12 ml Ethanol und 15 ml Toluol wird bei 10—15° unter Rühren bis zur Sättigung trockenes HCl-Gas eingeleitet. Nach 15 Stunden werden die Lösungsmittel entfernt und der Rückstand mit Methyl-tert.-butylether gerührt. Der entstandene Niederschlag wird nach dem Trocknen in 30 ml Ethanol aufgenommen und bei 10° mit einer Lösung von 10 g Ammoniak in 40 ml Ethanol versetzt. Nach 14 Stunden Analog werden hergestellt:

p-(4-Cyan-4-hexylcyclohexyl)-benzamidinhydrochlorid
p-(4-Cyan-4-pentylcyclohexyl)-benzamidinhydrochlorid
p-(4-Cyan-4-butylcyclohexyl)-benzamidinhydrochlorid
p-(4-Cyan-4-propylcyclohexyl)-benzamidinhydrochlorid
p-(4-Cyan-4-octylcyclohexyl)-benzamidinhydrochlorid
p-(4-Cyan-4-nonylcyclohexyl)-benzamidinhydrochlorid
p-(4-Cyan-4-decylcyclohexyl)-benzamidinhydrochlorid
p-[p-(4-Cyan-4-hexylcyclohexyl)-phenyl]-benzamidinhydrochlorid
p-[p-(4-Cyan-4-pentylcyclohexyl)-phenyl]-benzamidinhydrochlorid
p-[p-(4-Cyan-4-butylcyclohexyl)-phenyl]-benzamidinhydrochlorid
p-[p-(4-Cyan-4-propylcyclohexyl)-phenyl]-benzamidinhydrochlorid
p-[p-(4-Cyan-4-octylcyclohexyl)-phenyl]-benzamidinhydrochlorid
p-[p-(4-Cyan-4-nonylcyclohexyl)-phenyl]-benzamidinhydrochlorid
p-[p-(4-Cyan-4-decylcyclohexyl)-phenyl]-benzamidinhydrochlorid

**Patentansprüche**

1. r-1-Cyan-1-R²-cis-4-R¹-cyclohexanverbindungen, worin R¹ —CH₂X, Amino, Amidino, Formyl, p-R³-Phenyl oder 4-R³-Biphenyl-4'-yl ist, wobei X hydroxy, Halogen oder eine Sulfonatgruppe und R³ Amino, Amidino, Formyl, Hydroxy, Carboxy, Alkanoyl mit 2 bis 12 C-Atomen oder —CH₂X, worin X die oben agegebene Bedeutung hat, bedeutet, und R² eine Alkylgruppe mit 1 bis 12 C-Atomen ist, worin auch eine oder mehrere nicht benachbarte und nicht ringständige CH₂-Gruppen durch —O— oder —CH=CH— und/oder eine oder mehrere CH₂-Gruppen durch —CF₂— und/oder —CHF— ersetzt sein können, sowie die reaktionsfähigen Derivate und die Säureadditionssalze der basischen Cyclohexanverbindungen.

2. Verbindungen nach Anspruch 1, worin R¹ p-Hydroxyphenyl oder 4-Hydroxybiphenyl-4'-yl ist.

3. Verfahren zur Herstellung von Verbindungen nach Anspruch 2, dadurch gekennzeichnet, daß man eine entsprechende p-Alkoxyphenyl- bzw. 4-Alkyloxybiphenyl-4'-yl-Verbindung einer Etherspaltung unterwirft.

4. Verwendung von Verbindungen nach Anspruch 1 als Zwischenprodukte bei der Herstellung von Flüssigkristallmaterialien.

**Revendications**

1. r-1-cyano-1-R²-cis-4-R¹-cyclohexanes dans lesquels R¹ représente —CH₂X, un groupe amino, amidino, formyle, p-R³-phényle ou 4-R³-biphényle-4'-yle, X représente un groupe hydroxy, un halogène ou un groupe sulfonate et R³ représente un groupe amino, amidino, formyle, hydroxy, carboxy, alcanoyle en C2—C12 ou —CH₂X dans lequel X a les significations indiquées ci-dessus, et R² représente un groupe alkyle en C1—C12, et dans lesquels un ou pluiseurs groupes CH₂ non voisins et none situées sur un cycle peuvent être remplacés par —O— ou —CH=CH— et/ou un ou plusieurs groupes CH₂ peuvent être remplacés par —CF₂— et/ou —CHF—, ainsi que les dérivés réactifs et les sels des cyclohexanes basiques formés par addition avec des acides.

## EP 0 259 346 B1

2. Composés selon la revendication 1, dans lesquels R¹ représente un groupe p-hydroxyphényle ou 4-hydroxybiphényle-4'-yle.

3. Procédé de préparation des composés selon la revendication 2, caractérisé en ce que l'on soumet un composé correspondant p-alkyloxyphénylique ou respectivement 4-alkyloxybiphényle-4-ylique à scission du groupe éther.

4. Utilisation des composés selon la revendication 1 en tant que produits intermédiaires de la préparation de matières à cristaux liquides.

**Claims**

1. An r-1-cyano-1-R²-cis-4-R¹-cyclohexane compound in which R¹ is —CH₂X, amino, amidino, formyl, p-R³-phenyl or 4-R³-biphenyl-4'-yl, where X is hydroxyl, halogen or a sulfonate group and R³ is amino, amidino, formyl, hydroxyl, carboxyl, alkanoyl having 2 to 12 C atoms or —CH₂X, in which X has the abovementioned meaning, and R² is an alkyl group having 1 to 12 C atoms in which one or more CH₂ groups which are non-neighboring and not attached to the ring can be replaced by —O— or —CH=CH—, and/or one or more CH₂ groups can be replaced by —CF₂— and/or —CHF—, and the reactive derivatives and the acid-addition salts of the basic cyclohexane compounds.

2. A compound as claimed in claim 1, in which R¹ isw p-hydroxphenyl or 4-hydroxybiphenyl-4'-yl.

3. A process for the preparation of compounds as claimed in claim 2, wherein an appropriate p-alkyloxyphenyl- or 4-alkyloxybiphenyl-4'-yl compound is subjected to ether cleavage.

4. The use of a compound as claimed in claim 1 as an intermediate in the preparation of liquid-crystal materials.

6